# EUROPEAN PATENT APPLICATION

(11) **EP 4 324 469 A1**
(43) Date of publication of application: **21.02.2024**
(21) Application number: 22933051.9
(22) Date of filing: 19.10.2022
(51) Int. Cl.: A61K 35/28, C12N 5/0775, A61P 19/02

(54) **USE OF BONE MARROW MESENCHYMAL STEM CELL-DERIVED EXOSOMES IN REPAIRING ARTICULAR CARTILAGE DEFICIENCY**

(30) Priority: 02.06.2022 CN 202210620951
(71) Applicant: Nantong University, Nantong, Jiangsu 226019 (CN)
(72) Inventor: SUN, Cheng, Nantong, Jiangsu 226019 (CN); GU, Xiaosong, Nantong, Jiangsu 226019 (CN); GONG, Leilei, Nantong, Jiangsu 226019 (CN); CONG, Meng, Nantong, Jiangsu 226019 (CN); YANG, Hongwei, Nantong, Jiangsu 226019 (CN); ZHANG, Yu, Nantong, Jiangsu 226019 (CN); LI, Meiyuan, Nantong, Jiangsu 226019 (CN); WANG, Xiaomin, Nantong, Jiangsu 226019 (CN); XU, Lai, Nantong, Jiangsu 226019 (CN)
(74) Representative: Proi World Intellectual Property GmbH
(86) International application number: PCT/CN2022/126040
(87) International publication number: WO 2023/179000

(57) **Abstract**

The invention belongs to the field of biological medicine and relates to a preparation method of bone marrow mesenchymal stem cell exosomes and an application of the bone marrow mesenchymal stem cell exosomes in repairing an articular cartilage defect. The bone marrow mesenchymal stem cell exosomes are generated by stimulating mesenchymal stem cells with a culture medium, and extracted from a culture solution after passage; the bone marrow mesenchymal stem cells are derived from bone marrow of ilium or femur. An extracellular vesicle prepared from bone marrow mesenchymal stem cells has typical characteristics of an exosome; the prepared exosomes can improve cartilage cell vitality and promote the activity of cartilage cell proliferation and migration; in a rabbit articular cartilage defect model, the prepared exosomes can significantly promote cartilage repair.

## Description

### BACKGROUND OF THE INVENTION

### 1. Technical Field

The present invention belongs to the field of biological medicine, and relates to a preparation method of bone marrow mesenchymal stem cell exosomes and an application of the bone marrow mesenchymal stem cell exosomes in promoting cartilage cell vitality, improving cartilage cell proliferation, enhancing cartilage cell migration, and repairing a cartilage defect.

### 2. Description of Related Art

Cartilage is composed of collagen, proteoglycan, glycosaminoglycan, glycoprotein, a few cells, and 60%-80% water. Cartilage cells and the surrounding extracellular matrix (ECM) together constitute a cartilage unit, which is regarded as a main structural, functional and metabolic unit of cartilage. In normal cartilage, a cartilage cell synthesizes collagen, proteoglycan, glycosaminoglycan and other substances to maintain cartilage stability or replace damaged cartilage. Cartilage plays an important role in bearing loads and reducing bone friction between joints in the human body. However, due to the lack of blood vessels or nerves in adult cartilage tissue, the self-repair ability of an injured cartilage tissue is limited. A cartilage defect is often closely related to a degenerative joint disease, such as degenerative arthritis, senile arthritis, and hypertrophic arthritis. A clinical treatment method for treating a degenerative joint disease includes non-surgical treatment and surgical treatment. The non-surgical treatment mainly involves oral administration of a non-steroid anti-inflammatory medicine, intra-articular injection of a medicine, and rehabilitation exercise. Though this can alleviate short-term pain and relieve a symptom, its long-term effect is unsatisfactory. The surgical treatment includes microfracture, drilling, joint replacement surgery, autologous cartilage cell implantation, juvenile allogeneic cartilage transplantation and autologous cartilage transplantation. However, due to limitations of these different surgical procedures, including the formation of subchondral fibrous cartilage, limited tissue availability, dysfunction caused by dedifferentiation of primary cartilage cells during expansion, and possible inflammation around the donor cartilage, new alternative treatment strategies for cartilage regeneration are urgently needed. The treatment of cartilage damage based on the differentiation potential of the stem cell has been clinically proven to have an enormous development potential. At the same time, in order to avoid constraints such as stem cell tumorigenicity, the extracellular vesicles released by stem cells, especially the paracrine effect produced by exosomes, are increasingly valued for repairing cartilage damage.

### BRIEF SUMMARY OF THE INVENTION

The purpose of the present invention is to provide bone marrow mesenchymal stem cell exosomes, and an application of the bone marrow mesenchymal stem cell exosomes in promoting cartilage cell vitality, improving cartilage cell proliferation, enhancing cartilage cell migration, and repairing a cartilage defect. The exosomes can improve cartilage cell vitality, promote the cell proliferation, and enhance the ability of cell migration. Exosome treatment can significantly promote the repair of damaged cartilage and significantly improve the regeneration of damaged cartilage.

An application of bone marrow mesenchymal stem cell exosomes in preparing a medicine for treating an articular cartilage disease, wherein the bone marrow mesenchymal stem cell exosomes are generated by stimulating mesenchymal stem cells with a culture medium, and extracted from a culture solution after passage; the bone marrow mesenchymal stem cells are derived from bone marrow of ilium or femur.

Furthermore, the application comprises any of the following:
an application in preparing a medicine for promoting cartilage cell vitality;
an application in preparing a medicine for promoting cartilage cell proliferation;
an application in preparing a medicine for promoting cartilage cell migration;
an application in preparing a medicine for repairing a cartilage defect.
Furthermore, the culture medium is a complete culture medium.

Furthermore, culture conditions for the bone marrow mesenchymal stem cells are 37°C and 5% CO₂.

Furthermore, the bone marrow is added to the complete culture medium, blown and mixed, and placed in an incubator for culturing; half solution is replaced on the third day, and all the solution is replaced on the sixth day; subsequently, the solution is replaced every two days until the cell grows to 80%-90%, passed to P1 generation; primary bone marrow mesenchymal stem cells are obtained through three passages.

Furthermore, when the cell grows to a logarithmic phase in a course of culturing the bone marrow mesenchymal stem cells, the culture medium is discarded, the cell is washed with PBS, and then cultured in a 2% vesicle free serum culture medium until the cell grows to 80%-90%; a supernatant is collected, and a filtrate is used for exosome extraction.

Furthermore, the bone marrow mesenchymal stem cell exosomes are filtered and collected by a membrane affinity centrifugal column.

Furthermore, the filtered supernatant is added to an equal volume of XBP buffer, mixed, and then added to the membrane affinity centrifugal column for treatment; the exosome adsorbed on the column is eluted with an XE buffer.

Furthermore, the 0.2µm membrane is used for filtration.

Furthermore, the filtered supernatant is mixed, added to the membrane affinity centrifugal column for centrifugal treatment, one minute per 500g.

### Advantageous Effects

The present invention provides an application of bone marrow mesenchymal stem cell exosomes in preparing a medicine for treating a joint cartilage disease, and the exosomes can improve cartilage cell vitality, promote the cell proliferation, and enhance the ability of cell migration. Exosome treatment can significantly promote the repair of damaged cartilage and significantly improve the regeneration of damaged cartilage.

### BRIEF DESCRIPTION OF THE SEVERAL VIEWS OF THE DRAWINGS

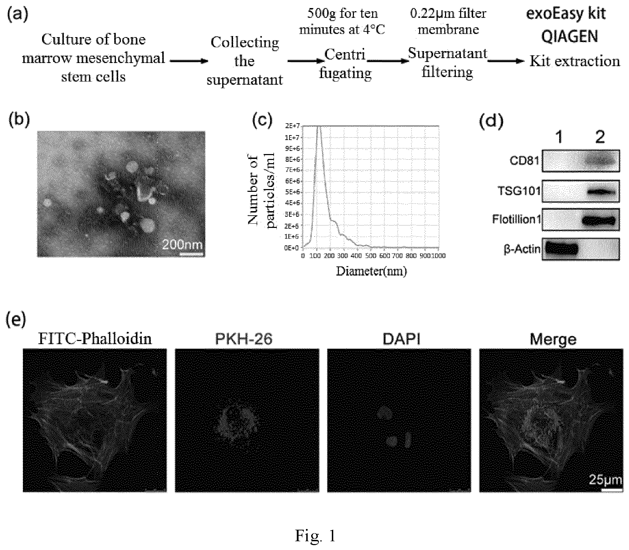

Fig. 1 shows the extraction and identification of bone marrow mesenchymal stem cell exosomes. (a) extraction process of bone marrow mesenchymal stem cell exosomes; (b) SEM photograph of bone marrow mesenchymal stem cell exosomes; (c) analysis of bone marrow mesenchymal stem cell exosomes by a nanoparticle tracker; (d) protein identification of bone marrow mesenchymal stem cell exosomes; 1: total protein sample of bone marrow mesenchymal stem cells; 2: protein sample of bone marrow mesenchymal stem cell exosomes. The protein expression is analyzed using western blot method; (e) internalizing identification of bone marrow mesenchymal stem cell exosomes. FITC-Phalloidin: fluorescein isothiocyanate-phalloidine, used for labeling cytoskeleton; PKH-26: red fluorescein, used for labeling exosomes; DAPI: 4',6-diamidino-2-phenylindole, used for labeling cell nucleus; Merge is a combination pattern.

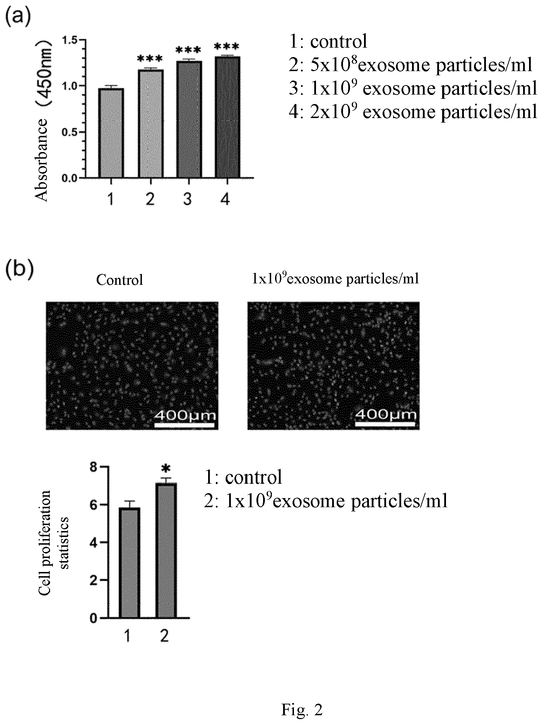

Fig. 2 shows bone marrow mesenchymal stem cell exosomes for promoting cartilage cell vitality and cell proliferation. (a) bone marrow mesenchymal stem cell exosomes improve cartilage cell vitality; (b) bone marrow mesenchymal stem cell exosomes promote cartilage cell proliferation. * p < 0.05, *** p < 0.001, Student's t-test analysis.

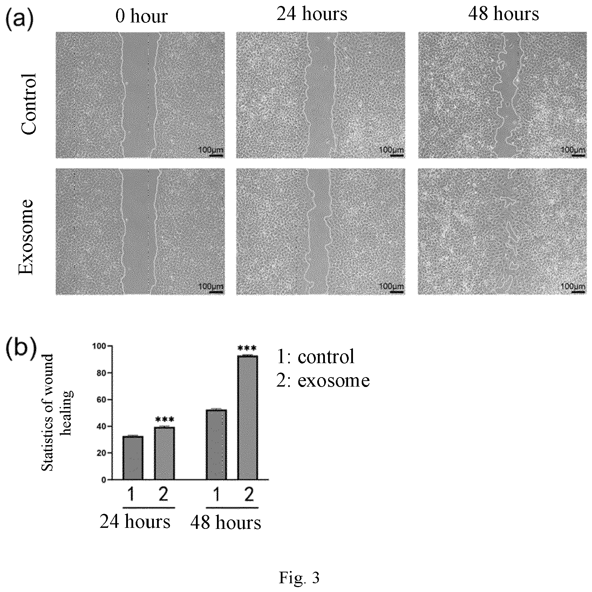

Fig. 3 shows bone marrow mesenchymal stem cell exosomes for promoting cartilage cell migration. (a) bone marrow mesenchymal stem cell exosomes are used for treating cartilage cells, and the ability of cell migration is tested by wound healing experiments; (b) quantitative analysis is carried out on the results of the wound healing experiments. *** p < 0.001, one-way ANOVA analysis.

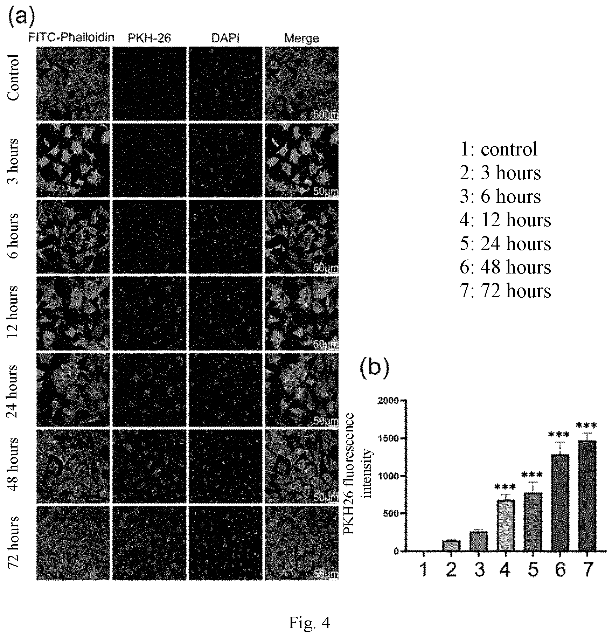

Fig. 4 shows the internalizing kinetic analysis of bone marrow mesenchymal stem cell exosomes in cartilage cells. (a) internalizing bone marrow mesenchymal stem cell exosomes. Bone marrow mesenchymal stem cell exosomes are labeled with PKH26, and the labeled exosomes are used for treating cartilage cells; signs of PKH26 in cells are tested at different times; (b) quantitative analysis is carried out on PHK26 intensity of bone marrow mesenchymal stem cell exosomes in cartilage cells at different times. FITC-Phalloidin: fluorescein isothiocyanate-phalloidine, used for labeling cytoskeleton; PKH-26: red fluorescein, used for labeling exosomes; DAPI: 4',6-diamidino-2-phenylindole, used for labeling cell nucleus; Merge is a combination pattern. *** p < 0.001, one-way ANOVA analysis.

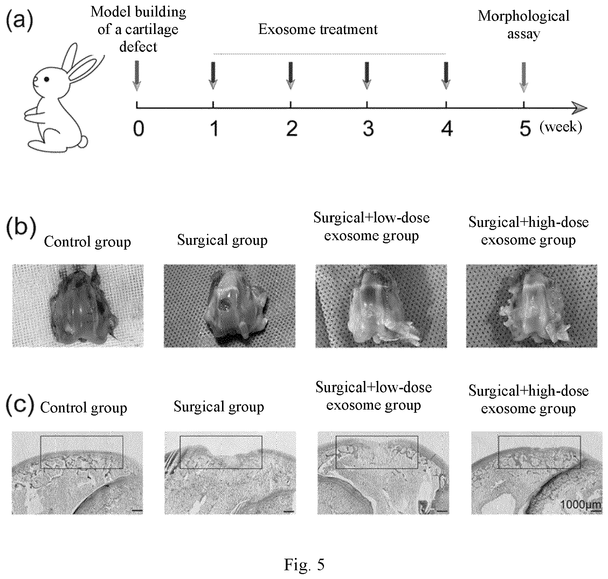

Fig. 5 shows bone marrow mesenchymal stem cell exosomes for promoting the cartilage repair. (a) experimental process; (b) external view of rabbit knee joint cartilage; (c) rabbit knee joint cartilage hematoxylin-eosin staining.

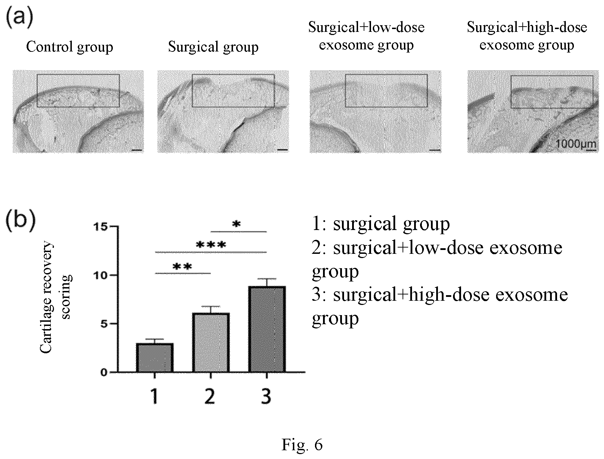

Fig. 6 shows bone marrow mesenchymal stem cell exosomes for promoting the cartilage regeneration. (a) Saf-O/Fast Green staining. (b) scoring is carried out on cartilage regeneration according to the scoring standard of the International Cartilage Society. * p < 0.05, ** p < 0.01, *** p < 0.001, one-way ANOVA analysis.

### DETAILED DESCRIPTION OF THE INVENTION

### I. Experimental steps

### 1. Extraction, culturing and identification of human bone marrow mesenchymal stem cell exosomes

Human bone marrow specimens are obtained from the ilium or femur of six normal adults (average age of 35 years old, ranging from 30 to 40 years old; male: three, female: three). 1ml human bone marrow extracted is placed in a 60mm culture dish, and 8ml complete culture medium is added, blown and mixed. The complete culture medium is DMEM cell culture solution, and added with 10% fetal calf serum and 1% penicillin and streptomycin. Afterwards, it is placed in a 5% CO₂ incubator at 37°C for culturing; half solution is replaced on the third day, and all the solution is replaced on the sixth day; subsequently, the solution is replaced every two days until the cell grows to 80%-90%, passing to P1 generation. After three passages, pure primary bone marrow mesenchymal stem cells are obtained, and P3-P5 cells are used for subsequent experiments.

### 2. Extraction of bone marrow mesenchymal stem cell exosomes

When the cell grows to a logarithmic phase, the culture medium is discarded, washed with PBS, then cultured in a 2% vesicle free serum culture medium for culture, and the 2% vesicle free serum culture medium is: the fetal calf serum is used for preparing the cell culture medium after vesicle removal through ultracentrifugation (100,000g, 16 hours, 4°C). The culture medium consists of: DMEM cell culture solution, containing 2% vesicle free fetal calf serum and 1% penicillin and streptomycin. When the cell grows to 80%-90%, a supernatant is collected, and filtered by a 0.2µM filter, and a filtrate is used for exosome extraction. The filtrate filtered by the 0.2µM filter is transferred to a new tube, and added with an equal volume of XBP buffer, and the tube is gently flipped five times for mixing the solution well. The mixture is added to exoEasy Kit column of an exosome kit, for centrifugal treatment, one minute per 500g. The effluent solution is discarded, and added with samples again until the mixture is completely filtered. The column is placed in the same collection tube, added with 10ml XWP buffer and centrifugated in 5,000g for five minutes, in order to remove any residual liquid from the column. The subnatant is discarded. The column is moved to a new collection tube, and the exosome adsorbed on the column is eluted with an XE buffer. The obtained exosome is preserved in a -80°C refrigerator for subsequent experiments.

### 3. Identification of an exosome

(1) An exosome is resuspended with PBS, and the particle size and concentration of the exosome are determined by nanoparticle tracking analyses (NTA).
(2) The exosome solution is deposited on a 300-mesh copper mesh coated with polymethyl vinyl acetate and carbon, placed at room temperature for three minutes, and then stained with 1.5% uranyl acetate. The mesh is imaged, observed, and photographed by TEM.
(3) Western Blot is used for testing the expression of bone marrow mesenchymal stem cell exosomes related protein, including CD81, Flotillion-1, TSG101.

### 4. Extraction and culture of a cartilage cell

Extraction of a cartilage cell: (1) taking cartilage slices from knee joints of four-week New Zealand rabbits; (2) cutting them into small pieces; washing with PBS three times; (3) digesting for 30 minutes with 0.25% trypsin; (4) discarding the supernatant, placing 0.2% type II collagenase in a 5% CO2 incubator at 37°C for digesting over night; (5) filtering with 200-mesh filter mesh, with centrifugal treatment, five minutes per 500g; (6) resuspending the precipitate with the complete cartilage cell culture medium, and then placing it in the incubator for culturing.

Cartilage cell passage: (1) replacing the solution with the complete culture medium every two days until the cell grows to 80%-90%; (2) washing with PBS twice, adding an appropriate volume of 0.25% pancreatin for digesting, the complete culture medium ending digestion and collection of cells, resuspending the culture plate after centrifugation; (3) obtaining purer primary cartilage cells after one passage, P1-P3 cartilage cells being used for subsequent experiments.

### 5. Test of cartilage cell vitality

In a 96-pore plate, cartilage cells are inoculated according to a density of 5×10³/ml. After inoculating for 4 hours, it is replaced with 2% vesicle free serum culture medium, and added with different concentrations of exosomes (5.0 × 10⁸/ml, 1.0 × 10⁹/ml2.0 × 10⁹/ml) for culturing for 24 hours. CCK-8 solution with the total volume of 1/10 culture medium is added. The culture plate is placed in an incubator at 37°C for incubation for 2-4 hours. The light absorption value at a wavelength of 450nm is determined.

### 6. Test of cartilage cell proliferation

In a 96-pore plate, cartilage cells are inoculated according to a density of 5 × 10³/ml. After inoculating for four hours, it is replaced with 2% vesicle free serum culture medium, and added with the prepared exosomes (1.0 × 10⁹/ml) for culturing for 24 hours. The culture medium is removed from each pore, and 50µl EdU is added to each pore for incubation for two hours. 100µl stationary liquid is added for fixing at the room temperature for fifteen minutes after discarding the culture medium and washing with PBS; 100µl 2 mg/ml glycine solution is added for neutralization reaction for five minutes; 100µl PBS is added for washing for five minutes; 100µl 0.5% TritonX-100 PBS is added for reaction for ten minutes and washing with PBS for five minutes. 100µl staining reaction solution is added for incubation in dark at the room temperature for 30 minutes; 100µl 0.5% TritonX-100 PBS is added for washing twice, ten minutes each time; 100µl Hoechst staining solution is added for incubation in dark at the room temperature in dark for fifteen minutes; the PBS decolorizing shaker is used for washing three times, 5-10 minutes each time. Cell images are captured using a fluorescence microscope EVOS FL Auto.

### 7. Cell wound healing test

The cell wound healing test is simulated by using an ibidi device: (1) inserting a two-hole device into a pre-coated small dish for a wound healing test; (2) preparing pre-treated cell suspension, i.e. resuspending cells using serum-free culture medium; adding cell suspension based on a concentration of 2×10⁵/ml each hole (100µl); (3) after 12 hours, gently removing the two-hole device with sterile tweezers and washing it with PBS once; (4) replacing the culture medium in the control group with 2% vesicle free serum culture medium, while replacing the culture medium in the treatment group with 2% vesicle free serum medium containing exosomes (1.0×10⁹/ml), and continuing to culture for 24 and 48 hours; (5) observing the cell migration process under a microscope and photographing.

### 8. Exosome internalizing test

Exosomes are labeled with PKH26, comprising the following process: (1) preparing exosome solution with a concentration of 1×10¹¹/ml (100µl); (2) adding exosomes into 250µl Diluent C, and gently mixing well; (3) taking a new EP tube, and adding 2µl PKH26 to 250µl Diluent C; (4) adding (2) to (3), and mixing; (5) leaving the tube at room temperature for 4 minutes, and mixing with a pipette every one minute; (6) adding an equal volume of 1% BSA to terminate staining; (7) concentrating the solution with Amicon Ultra Centrifugal Filters for use; (8) diluting the concentrated exosome with a serum-free culture medium, and finally preparing a solution containing 1.0×10⁹/ml exosome; (9) after four hours of cartilage cell inoculation, adding PKH26 labeled exosomes for co-culturing for 3, 6, 12, 24, 48, and 72 hours; (10) fixing cells with 4%PFA after treatment; (11) washing with PBS twice, ten minutes each time; (12) adding 100µl DAPI staining solution, incubating in dark at the room temperature for fifteen minutes; (13) observing and photographing under a confocal microscopy.

### 9. Model building of a cartilage defect

New Zealand rabbits are provided by the Animal Center of Nantong University. They are divided into four groups: control group, surgical group, surgical+low-dose exosome group, surgical+high-dose exosome group, with 6 rabbits in each group. Metomidine (20µg/kg) is intramuscularly injected, and ketamine (5mg/kg) and buprenorphine (0.03mg/kg) are administered in combination. Isoflurane is used for anesthesia, and an osteochondral defect with a diameter of 4mm and a depth of 3mm is created in the femoral trochlear groove of the knee joint using a drill bit in the left hind limb of the rabbit. The incision is closed and sutured layer by layer. Penicillin (50,000 units/kg) is intramuscularly injected for three consecutive days to prevent infection, with 2 mg/kg tramadol for analgesia. Add different concentrations (low dose: 1.0 × 10¹⁰/ml; high dose: 5.0 × 10¹⁰/ml) of 300µl exosomes into the joint cavity once a week for a total of four times.

### 10. Paraffin slice

At the end of the test, the rabbits are euthanized, and the complete knee joint is removed; it is fixed with paraformaldehyde for 24 hours, and decalcified with 10% EDTA (pH 7.4) for 2-3 months; the knee joint is embedded in paraffin and cut into 5µm thick slices. Paraffin slices are dewaxed to water: The slices are placed slices in xylene (I) for 20 minutes - xylene (II) for 20 minutes - anhydrous ethanol (I) for five minutes - anhydrous ethanol (II) for five minutes -75% ethanol for five minutes, and washed with tap water.

### 11. Staining with hematoxylin and eosin (H&E)

It is stained with hematoxylin solution (60°C) for 30-60 seconds, and the hematoxylin solution is washed with running water for 5-10 seconds, with 1% hydrochloric acid ethanol for 1-3 seconds, slightly washed with running water for 1-2 seconds, blued with bluing solution for 5-10 seconds, washed with running water for 15-30 seconds, stained with 0.5% eosin solution for 30-60 seconds, slightly washed with distilled water for 1-2 seconds, 80% ethanol for 1-2 seconds, 95% ethanol for 1-2 seconds, anhydrous ethanol for 1-2 seconds, xylene (I) for 2-3 seconds, xylene (II) for 2-3 seconds, and sealed with neutral gum.

### 12. Staining with Safranin O/Fast Green

Staining with fast green: placing the slices in the fast green dye liquor for 5-10 minutes, removing excess dye liquor with water until the cartilage is colorless, soaking in the differentiation solution slightly, and washing with three-color distilled water. Staining with Safranin O: placing the slices in the Safranin O dye liquor for 15-30 seconds, and dewatering quickly with anhydrous ethanol. Transparent sealing: conducting transparent treatment on clean xylene for five minutes, and sealing with neutral gum; examining under a microscope, and acquiring images for analysis.

### 13. Cartilage regeneration scoring

According to the International Cartilage Repair Society (ICRS), cartilage damage is scored.

The bone marrow mesenchymal stem cell culture supernatant is collected for exosome extraction. The cell fragments in the supernatant will be removed by centrifugation, and the obtained supernatant will be filtered and then subjected to exosomes extraction using a commercial exosome extraction kit (Fig. 1a). The obtained exosomes are identified accordingly. Firstly, the extracted exosome is observed with an electron microscope, and it is found that, the extracted exosome exhibits a typical exosome appearance, i.e., cup-shaped vesicle with double membranes (Fig. 1b); then, analysis with a nanoparticle tracker shows that, the extracted exosome has an average diameter of 131.20nm and a concentration of 1.2 x 10¹¹ particles/ml (Fig. 1c); protein assay shows that the extracted exosomes express typical proteins, including CD81, TSG10, Flotillion 1, but do not express Actin (Fig. 1d); finally, the internalizing ability of the extracted exosomes is tested. The results show that, when exosomes are co-cultured with cartilage cells, it is found that exosomes can be internalized (Fig. 1e). The above results indicate that the extracellular vesicles extracted from the supernatant of bone marrow mesenchymal stem cells are exosomes.

Functional experiments show that the extracted exosomes can improve cartilage cell vitality (Fig. 2a), promote the cell proliferation (Fig. 2b), and enhance the ability of cell migration (Figs. 3a, b). To determine the in vivo treatment method, exosome internalizing kinetic tests are conducted. The results show that, exosomes can be observed in the cells after co-culturing with cartilage cells for 3 hours. As the treatment time goes by, the number of exosomes in the cells gradually increases, and the peak of internalization occurs at 72 hours after treatment (Figs. 4a, b. In vivo experiments are conducted using a rabbit's hind knee joint cartilage defect model. The prepared exosomes are injected into the joint cavity four times, followed by morphological analysis of cartilage repair (Fig. 5a). Observing the gross morphology of the rabbit's knee joint, it is found that the cartilage defect of the rabbit's knee joint in the surgical group is still clearly visible; after exosome treatment, cartilage defects are significantly restored, and the high-dose group shows better recovery (Fig. 5b). The results of staining with hematoxylin and eosin also show that, exosome treatment can significantly promote repair of damaged cartilage (Fig. 5c). The results of staining with Safranin O/Fast Green show that, the knee joint cartilage defects in the rabbits of the surgical group are not improved, but exosome treatment significantly can significantly improve the regeneration of the damaged cartilage (Fig. 6a); based on the standards established by the International Cartilage Society for scoring, exosome treatment can significantly increase this score, with the high-dose exosome group scoring better than the low-dose group (Fig. 6b).

## Claims

1. An application of bone marrow mesenchymal stem cell exosomes in preparing a medicine for treating an articular cartilage disease, wherein the bone marrow mesenchymal stem cell exosomes are generated by stimulating mesenchymal stem cells with a culture medium, and extracted from a culture solution after passage; the bone marrow mesenchymal stem cells are derived from bone marrow of ilium or femur.

2. The application according to claim 1, wherein the application comprises any of the following:
an application in preparing a medicine for promoting cartilage cell vitality;
an application in preparing a medicine for promoting cartilage cell proliferation;
an application in preparing a medicine for promoting cartilage cell migration;
an application in preparing a medicine for repairing a cartilage defect.

3. The application according to claim 1, wherein the culture medium is a complete culture medium.

4. The application according to claim 1, wherein culture conditions for the bone marrow mesenchymal stem cells are 37°C and 5% CO₂.

5. The application according to claim 1, wherein the bone marrow is added to a complete culture medium, blown and mixed, and placed in an incubator for culturing; half solution is replaced on the third day, and all the solution is replaced on the sixth day; subsequently, the solution is replaced every two days until the cell grows to 80%-90% and is passed to P1 generation; primary bone marrow mesenchymal stem cells are obtained through three passages.

6. The application according to claim 1, wherein when the cell grows to a logarithmic phase in a course of culturing the bone marrow mesenchymal stem cells, the culture medium is discarded, the cell is washed with PBS, and then cultured in a 2% vesicle free serum culture medium until the cell grows to 80%-90%; a supernatant is collected, and a filtrate is used for exosome extraction.

7. The application according to claim 1, wherein the bone marrow mesenchymal stem cell exosomes are filtered and collected by a membrane affinity centrifugal column.

8. The application according to claim 6, wherein an equal volume of XBP buffer is added to the filtered supernatant, mixed, and then added to the membrane affinity centrifugal column for treatment; an exosome adsorbed on the column is eluted with an XE buffer.

9. The application according to claim 8, wherein a 0.2µm membrane is used for filtration.

10. The application according to claim 8, wherein the filtered supernatant that is mixed is added to the membrane affinity centrifugal column for centrifugal treatment, one minute per 500g.
